(19) Europäisches Patentamt
European Patent Office
Office européen des brevets

(11) EP 2 631 233 A1

(12) # EUROPEAN PATENT APPLICATION
published in accordance with Art. 153(4) EPC

(43) Date of publication:
**28.08.2013 Bulletin 2013/35**

(21) Application number: **11834469.6**

(22) Date of filing: **21.10.2011**

(51) Int Cl.:
*C07D 401/12* (2006.01) *A61K 31/496* (2006.01)
*A61K 31/506* (2006.01) *A61P 5/26* (2006.01)
*A61P 35/00* (2006.01) *C07D 403/12* (2006.01)

(86) International application number:
**PCT/JP2011/074261**

(87) International publication number:
**WO 2012/053630 (26.04.2012 Gazette 2012/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **22.10.2010 JP 2010237242**

(71) Applicant: **Astellas Pharma Inc.**
**Tokyo 103-8411 (JP)**

(72) Inventors:
- **IDEYAMA, Yukitaka**
  **Tokyo 103-8411 (JP)**
- **KUROMITSU, Sadao**
  **Tokyo 103-8411 (JP)**
- **FURUTANI, Takashi**
  **Tokyo 103-8411 (JP)**
- **TAKEDA, Masayoshi**
  **Tokyo 103-8411 (JP)**
- **KONAGAI, Satoshi**
  **Tokyo 103-8411 (JP)**
- **YAMADA, Tomohiro**
  **Tokyo 103-8411 (JP)**
- **TANIGUCHI, Nobuaki**
  **Tokyo 103-8411 (JP)**
- **KONDOH, Yutaka**
  **Tokyo 103-8411 (JP)**
- **HIRANO, Masaaki**
  **Tokyo 103-8411 (JP)**
- **WATANABE, Kazushi**
  **Tokyo 103-8411 (JP)**
- **SUGANE, Takashi**
  **Tokyo 103-8411 (JP)**
- **KAKEFUDA, Akio**
  **Tokyo 103-8411 (JP)**

(74) Representative: **HOFFMANN EITLE**
**Patent- und Rechtsanwälte**
**Arabellastrasse 4**
**81925 München (DE)**

(54) **ANTAGONIST FOR MUTATED ANDROGEN RECEPTOR**

(57) [Problem] An object of the present invention is to provide a novel anticancer drug which is useful for treating prostate cancer accompanying androgen receptor mutation [Means for Solution] The present inventors conducted thorough research on mutant androgen-related diseases for which the traditional anti-androgen drugs become ineffective. As a result, they found that the compound, which is an active ingredient of the pharmaceutical composition of the present invention, exhibits an inhibitory action against transcriptional activation in a human mutant androgen receptor (AR), and has an excellent antitumor action in a human prostate cancer-bearing mouse, thereby completing the present invention. Accordingly, the compound, which is an active ingredient of the pharmaceutical composition of the present invention, is useful for a series of androgen receptor-related diseases including prostate cancer.

EP 2 631 233 A1

## Description

Technical Field

The present invention relates to a pharmaceutical composition, particularly, an anticancer drug which is useful for treating prostate cancer accompanying androgen receptor mutation (AR mutation).

Background Art

The prostate is an organ unique to males, and among diseases of the prostate, prostate cancer has become a serious social issue. The prostate cancer is one of the most frequently occurring malignant tumors of males in Western countries, and accounts for about 20% of the cause of cancer deaths of males. Moreover, since the incidence of the prostate cancer increases with age, aging is an important factor inducing the prostate cancer. For the further aging society in the future, research on the prostate cancer is considered to have great significance.
Since most (about 80% or more) of prostate cancer shows androgen-dependent growth, GnRH (Gonadotropin releasing hormone) analogues or antagonists for an androgen receptor (hereinafter, described as "AR") are used for treating patients with advanced prostate cancer. These potently inhibit the growth of prostate cancer in the initial stage of administration. However, there is a big problem that the disease recurs within several years with such treatment. As one of the signs of recurrence a phenomenon called AWS (Anti-androgen withdrawal syndrome) (phenomenon in which an AR antagonist as an agonist) is recognized (J. Cell. Biochem 2007, 91, 3-12). That is, in patients with prostate cancer treated with both the GnRH analogue and the AR antagonist, the blood PAS (Prostate Specific Antigen) level, which is an index of the prostate cancer, is reduced for a certain period from the beginning of the administration, but the PSA level increases in some cases when the drugs are continuously administered. In such case, the PSA level is reduced by stopping the administration of the AR antagonist, and this phenomenon is called AWS. AWS is seen in about 30% of patients administered with the AR antagonist, and AR mutation is considered to be the main cause. Currently, bicalutamide is almost exclusively used as the AR antagonist. It is reported that W741C mutant AR is detected in the patients with prostate cancer for whom bicalutamide is ineffective, and that if the W741C mutant AR is introduced to PC-3 which is a prostate cancer cell not having AR, bicalutamide acts as an agonist. These facts imply that W741C mutant AR is involved in AWS of bicalutamide. In addition, as an AR antagonist other than bicalutamide, there is flutamide. However, currently, flutamide is almost not used because the drug causes disorder in the liver and digestive tract and is poor in compliance due to the three times of administration per day. Hydroxyflutamide which is an active metabolite of flutamide is known to act as an agonist in T877A mutant AR.

For the above reasons, there is a strong demand for a novel AR antagonist having potent antagonistic action against not only wild type ARs (normal ARs) but also mutant ARs (particularly, W741C mutant AR, that is, a bicalutamide-resistant mutant AR, and T877A mutant AR, that is, flutamide-resistant mutant AR).

Incidentally, the prostate cancer can be divided into the following stages (1) to (5) according to the development of the pathological condition and therapeutic approach corresponding thereto.

(1) Diagnosis of advanced prostate cancer
(2) Starting hormone therapy: a GnRH analogue and an AR antagonist (generally, bicalutamide) are used.
(3) The AR antagonist becomes ineffective since the patient exhibits resistance to the AR antagonist (generally, bicalutamide).
(4) The use of Docetaxel as a chemotherapy agent is started.
(5) Docetaxel also becomes ineffective since the patient exhibits resistance to it. In the above stages, the prostate cancer of stage (1) is called "Hormone-naive prostate cancer", and the prostate cancer of stage (2) is called "Hormone sensitive prostate cancer (HSPC)".
Moreover, the prostate cancer in the respective stages (3) to (5) is called "Castration resistant prostate cancer (CRPC)". Among the castration resistant prostate cancers, stage (3) corresponds to "chemo-naive CRPC", and stage (5) corresponds to "chemo-failure CRPC".

Further, the mutant ARs are classified into various types according to the site and the amino acid type of mutation in an amino acid sequence encoding the AR receptor protein. Regarding this point, for example, Non-Patent Documents 1 to 4 can be referred to, and at least 44 types of AR mutation have been reported (Non-Patent Document 3). Among these, the AR mutation caused by the administration of bicalutamide, which is a fist choice drug of the AR antagonist, forms a W741C mutant AR.

By the way, as an AR antagonist effective for mutant ARs, the following compound (a) has been reported (Patent Document 1).

[Chem. 1]

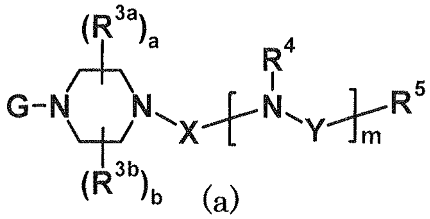

(a)

(In the formula, G represents bicyclic or tricyclic aryl or heteroaryl, 5-membered heteroaryl, pyridyl, or substituted phenyl having a substituent different from the compound of the present invention. See the corresponding gazette for more detail.) Patent Document 1 discloses the effectiveness of the compound of Formula (a) on T877A, L701H, and H874Y mutant ARs, but the document does not disclose or suggest the effectiveness on other types of mutant ARs.

In addition, as an AR antagonist, a compound represented by the following Formula (b) has been reported (Patent Document 2).

[Chem. 2]

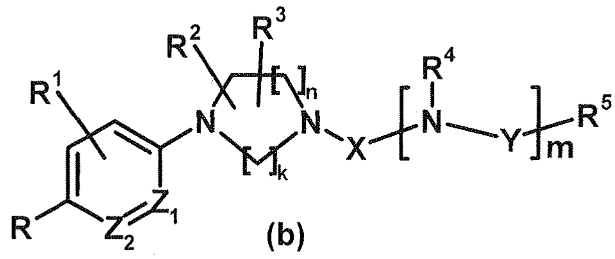

(b)

(In the formula, R represents a cyano or nitro group; $Z_1$ and $Z_2$ are the same as or different from each other and represent CH or a nitrogen atom; and X represents -C(=O)-, -C(=S)-, or -S(O)$_2$-. See the corresponding gazette for more detail.) Patent Document 2 relates to an AR antagonist and an agent for treating prostate cancer in which the AR is a growth factor, prostatism, virilizing syndrome, hirsutism, and the like. However, the document does not have disclosure or suggestion relating to the antagonistic action against a mutant AR.

Moreover, as an AR antagonist, a compound represented by the following Formula (c) has been reported (Patent Document 3).

[Chem. 3]

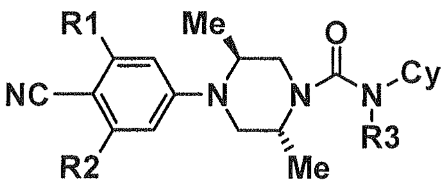

(c)

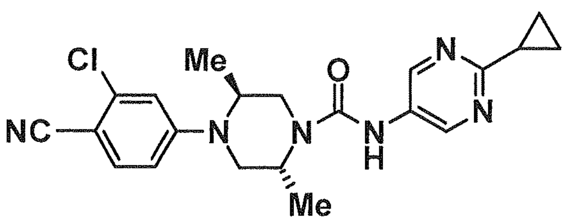

Ex 3-9
(Compound A)

(In the formula, Cy represents various types of substituted aryl or substituted heteroaryl. See the corresponding gazette for more detail.)

The patent document discloses a compound of Example 3-9 (Ex 3-9) having the chemical structure shown in the above drawing, and the compound will be called Compound A hereinafter.

Patent Document 3 relates to an AR antagonist and an agent for treating prostate cancer in which the AR acts as a factor of exacerbation, prostatism, virilizing syndrome, hirsutism, and the like. However, the document does not have disclosure or suggestion relating to the antagonistic action against a mutant AR.

Furthermore, as an agent for treating prostate cancer accompanying a mutant AR, for example, compounds (d) to (h) represented by the following formulae have been reported (Patent Documents 4 to 8).

[Chem. 4]

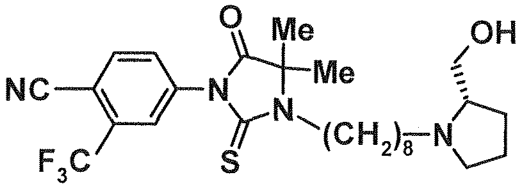

(d)

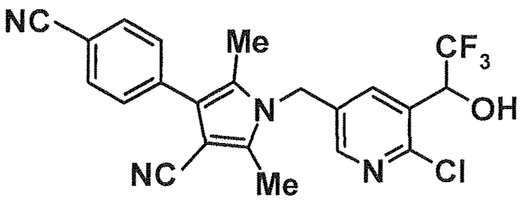

(e)

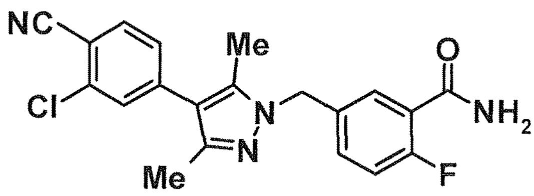

(f)

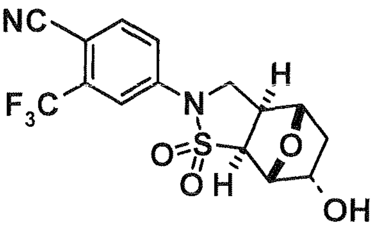

(g)

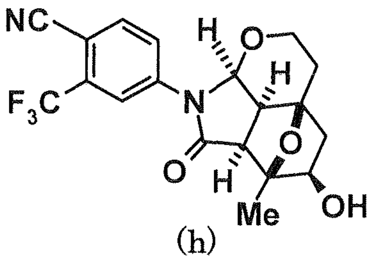

(h)

Patent Document 1 and Patent Documents 4 to 8 disclose various compounds effective for mutant ARs. However, the documents do not have disclosure or suggestion that when an AR antagonist which is effective for a wild type AR becomes ineffective due to a certain type of AR mutation, how to change the chemical structure of the AR antagonist which becomes ineffective in order to obtain a compound having an effective anticancer action against a specific mutant AR, that is, an antagonist for mutant AR.

Patent Document

Patent Document 1: Pamphlet of International Publication WO 2005/040136
Patent Document 2: Pamphlet of International Publication WO 2000/017163
Patent Document 3: Pamphlet of International Publication WO 2004/007471
Patent Document 4: US Publication No. 2005/0159468 A1
Patent Document 5: Pamphlet of International Publication WO 2006/064944
Patent Document 6: Pamphlet of International Publication WO 2009/028543
Patent Document 7: Pamphlet of International Publication WO 2009/059077
Patent Document 8: Pamphlet of International Publication WO 2009/003077

Non-Patent Document

Non-Patent Document 1: Cancer Research, 2003, Vol. 63, pp 149-153
Non-Patent Document 2: Journal of Cellular Biochemistry, 2004, Vol. 91, pp 3-12
Non-Patent Document 3: Cancer Research, 2002, Vol. 62, pp 1496-1502
Non-Patent Document 4: Science, 2009, Vol. 324, pp 787-790

Disclosure of Invention

Problems to Be Solved by the Present Invention

An object of the present invention is to provide a novel drug for treating prostate cancer accompanying AR mutation, particularly, castration resistant prostate cancer (CRPC) resistant to bicalutamide or flutamide.

Means for Solving the Problems

The present inventors conducted thorough research regarding the effect of various N-phenyl-(2R,5S)-dimethylpiperazine compounds on mutant ARs. As a result, they found that a specific N-phenyl-(2R,5S)-dimethylpiperazine compound exhibits excellent receptor antagonism against mutant ARs. They also found that the compound exhibits excellent antitumor effect on the prostate cancer accompanying AR mutation, and discovered that the compound can be used as a drug for excellently preventing or treating castration resistant prostate cancer, thereby completing the present invention.
That is, they found that some of the compounds disclosed in Patent Documents 2 and 3 are effective for treating the prostate cancer accompanying AR mutation, and the other compounds are ineffective for treating the prostate cancer accompanying AR mutation. Accordingly, the present invention relates to the use of the compounds that is newly found only in the above some compounds useful for treating prostate cancer accompanying AR mutation, including the use for treating prostate cancer accompanying AR mutation, particularly, the use for treating castration resistant prostate cancer (CRPC) resistant to bicalutamide or flutamide.
That is, the present invention contains the following:

(1) A pharmaceutical composition for treating castration resistant prostate cancer(CRPC), which comprises, as an active ingredient, a compound or a pharmaceutically acceptable salt thereof selected from a group consisting of (2R,5S)-4-(3-chloro-4-cyanophenyl)-N-(2-cyclopropylpyrimidin-5-yl)-2,5-dimethylpiperazine-1-carboxamide, (2R, 5S)-4-(4-cyano-3-methoxyphenyl)-N-(2,6-dimethylpyrimidin-4-yl)-2,5-dimethylpiperazine-1-carboxamide, (2R,5S)-4-(3-chloro-4-cyanophenyl)-N-(2-chloro-6-methylpyridin-4-yl)-2,5-dimethylpiperazine-1-carboxamide, (2R,5S)-4-(3-chloro-4-cyanophenyl)-N-(2-methoxy-6-methylpyridin-4-yl)-2,5-dimethylpiperazine-1-carboxamide, (2R,5S)-4-(3-bromo-4-cyanophenyl)-N-(2-methoxypyrimidin-5-yl)-2,5-dimethylpiperazine-1-carboxamide, (2R,5S)-4-(3-bromo-4-cyanophenyl)-N-(2-methoxy-6-methylpyridin-4-yl)-2,5-dimethylpiperazine-1-carboxamide, and (2R,5S)-4-(3-chloro-4-cyanophenyl)-2,5-dimethyl-N-[2-(piperidin-1-yl)pyrimidin-5-yl]piperazine-1-carboxamide, and pharmaceutically acceptable salts thereof.
(2) The pharmaceutical composition according to above (1), wherein the castration resistant prostate cancer (CRPC) is castration resistant prostate cancer resistant to bicalutamide.
(3) The pharmaceutical composition according to above (1), wherein the castration resistant prostate cancer (CRPC) is castration resistant prostate cancer resistant to flutamide.
(4) A compound or a pharmaceutically acceptable salt thereof selected from a group consisting of (2R,5S)-4-(3-chloro-4-cyanophenyl)-N-(2-cyclopropylpyrimidin-5-yl)-2,5-dimethylpiperazine-1-carboxamide monohydrate,
(2R,5S)-4-(4-cyano-3-methoxyphenyl)-N-(2,6-dimethylpyrimidin-4-yl)-2,5-dimethylpiperazine-1-carboxamide,
(2R,5S)-4-(3-chloro-4-cyanophenyl)-N-(2-chloro-6-methylpyridin-4-yl)-2,5-dimethylpiperazine-1-carboxamide,
(2R,5S)-4-(3-chloro-4-cyanophenyl)-N-(2-methoxy-6-methylpyridin-4-yl)-2,5-dimethylpiperazine-1-carboxamide,
(2R,5S)-4-(3-bromo-4-cyanophenyl)-N-(2-methoxypyrimidin-5-yl)-2,5-dimethylpiperazine-1-carboxamide,
(2R,5S)-4-(3-bromo-4-cyanophenyl)-N-(2-methoxy-6-methylpyridin-4-yl)-2,5-dimethylpiperazine-1-carboxamide,
and
(2R,5 S)-4-(3 -chloro-4-cyanophenyl)-2,5 -dimethyl-N-[2-(piperidin-1-yl)pyrimidin-5-yl]piperazine-1-carboxamide,
and
pharmaceutically acceptable salts thereof.
(5) The compound or a pharmaceutically acceptable salt thereof according to above (4), wherein the compound is (2R,5S)-4-(3-chloro-4-cyanophenyl)-N-(2-cyclopropylpyrimidin-5-yl)-2,5-dimethylpiperazine-1-carboxamide monohydrate.
(6) A compound or a pharmaceutically acceptable salt thereof for treating castration resistant prostate cancer (CRPC), which is selected from a group consisting of (2R,5S)-4-(3-chloro-4-cyanophenyl)-N-(2-cycyopropylpyrimidin-5-yl)-2,5-dimethylpiperazine-1-carboxamide,
(2R,5S)-4-(4-cyano-3-methoxyphenyl)-N-(2,6-dimethylpyrimidin-4-yl)-2,5-dimethylpiperazine-1-carboxamide,
(2R,5S)-4-(3-chloro-4-cyanophenyl)-N-(2-chloro-6-methylpyridin-4-yl)-2,5-dimethylpiperazine-1-carboxamide,
(2R,5S)-4-(3-chloro-4-cyanophenyl)-N-(2-methoxy-6-methylpyridin-4-yl)-2,5-dimethylpiperazine-1-carboxamide,
(2R,5S)-4-(3-bromo-4-cyanophenyl)-N-(2-methoxypyrimidin-5-yl)-2,5-dimethylpiperazine-1-carboxamide,

(2R,5S)-4-(3-bromo-4-cyanophenyl)-N-(2-methoxy-6-methylpyridin-4-yl)-2,5-dimethylpiperazine-1-carboxamide, and
(2R,5S)-4-(3-chloro-4-cyanophenyl)-2,5-dimethyl-N-[2-(piperidin-1-yl)pyrimidin-5-yl]piperazine-1-carboxamide, and
pharmaceutically acceptable salts thereof.
(7) Use of a compound or a pharmaceutically acceptable salt thereof for manufacturing a pharmaceutical composition for treating castration resistant prostate cancer (CRPC), wherein the compound or a pharmaceutically acceptable salt thereof is selected from a group consisting of (2R,5S)-4-(3-chloro-4-cyanophenyl)-N-(2-cyclopropylpyrimidin-5-yl)-2,5-dimethylpiperazine-1-carboxamide,
(2R,5S)-4-(4-cyano-3-methoxyphenyl)-N-(2,6-dimethylpyrimidin-4-yl)-2,5-dimethylpiperazine-1-carboxamide,
(2R,5S)-4-(3-chloro-4-cyanophenyl)-N-(2-chloro-6-methylpyridin-4-yl)-2,5-dimethylpiperazine-1-carboxamide,
(2R,5S)-4-(3-chloro-4-cyanophenyl)-N-(2-methoxy-6-methylpyridin-4-yl)-2,5-dimethylpiperazine-1-carboxamide,
(2R,5S)-4-(3-bromo-4-cyanophenyl)-N-(2-methoxypyrimidin-5-yl)-2,5-dimethylpiperazine-1-carboxamide,
(2R,5S)-4-(3-bromo-4-cyanophenyl)-N-(2-methoxy-6-methylpyridin-4-yl)-2,5-dimethylpiperazine-1-carboxamide, and
(2R,5S)-4-(3-chloro-4-cyanophenyl)-2,5-dimethyl-N-[2-(piperidin-1-yl)pyrimidin-5-yl]piperazine-1-carboxamide, and
pharmaceutically acceptable salts thereof.
(8) Use of a compound or a pharmaceutically acceptable salt thereof for treating castration resistant prostate cancer (CRPC), wherein the compound or a pharmaceutically acceptable salt thereof is selected from a group consisting of (2R,5S)-4-(3-chloro-4-cyanophenyl)-N-(2-cyclopropylpyrimidin-5-yl)-2,5-dimethylpiperazine-1-carboxamide,
(2R,5S)-4-(4-cyano-3-methoxyphenyl)-N-(2,6-dimethylpyrimidin-4-yl)-2,5-dimethylpiperazine-1-carboxamide,
(2R,5S)-4-(3-chloro-4-cyanophenyl)-N-(2-chloro-6-methylpyridin-4-yl)-2,5-dimethylpiperazine-1-carboxamide,
(2R,5S)-4-(3-chloro-4-cyanophenyl)-N-(2-methoxy-6-methylpyridin-4-yl)-2,5-dimethylpiperazine-1-carboxamide,
(2R,5S)-4-(3-bromo-4-cyanophenyl)-N-(2-methoxypyrimidin-5-yl)-2,5-dimethylpiperazine-1-carboxamide,
(2R,5S)-4-(3-bromo-4-cyanophenyl)-N-(2-methoxy-6-methylpyridin-4-yl)-2,5-dimethylpiperazine-1-carboxamide, and
(2R,5S)-4-(3-chloro-4-cyanophenyl)-2,5-dimethyl-N-[2-(piperidin-1-yl)pyrimidin-5-yl]piperazine-1-carboxamide, and
pharmaceutically acceptable salts thereof.
(9) A method of treating castration resistant prostate cancer (CRPC), which comprises administering to a subject an effective amount of a compound or a pharmaceutically acceptable salt thereof selected from a group consisting of (2R,5S)-4-(3-chloro-4-cyanophenyl)-N-(2-cyclopropylpyrimidin-5-yl)-2,5-dimethylpiperazine-1-carboxamide,
(2R,5S)-4-(4-cyano-3-methoxyphenyl)-N-(2,6-dimethylpyrimidin-4-yl)-2,5-dimethylpiperazine-1-carboxamide;
(2R,5S)-4-(3-chloro-4-cyanophenyl)-N-(2-chloro-6-methylpyridin-4-yl)-2,5-dimethylpiperazine-1-carboxamide,
(2R,5S)-4-(3-chloro-4-cyanophenyl)-N-(2-methoxy-6-methylpyridin-4-yl)-2,5-dimethylpiperazine-1-carboxamide,
(2R,5S)-4-(3-bromo-4-cyanophenyl)-N-(2-methoxypyrimidin-5-yl)-2,5-dimethylpiperazine-1-carboxamide,
(2R,5S)-4-(3-bromo-4-cyanophenyl)-N-(2-methoxy-6-methylpyridin-4-yl)-2,5-dimethylpiperazine-1-carboxamide, and
(2R,5S)-4-(3-chloro-4-cyanophenyl)-2,5-dimethyl-N-[2-(piperidin-1-yl)pyrimidin-5-yl]piperazine-1-carboxamide, and
pharmaceutically acceptable salts thereof.
In addition, the "subject" refers to a human being or other mammals that require the prevention or treatment, and as another embodiment, the subject refers to a human being who requires the prevention or treatment.

A compound which is an active ingredient of the pharmaceutical composition of the present invention is, for example, (2R,5S)-4-(3-chloro-4-cyanophenyl)-N-(2-cyclopropylpyrimidin-5-yl)-2,5-dimethylpiperazine-1-carboxamide monohydrate;
in another embodiment, (2R,5S)-4-(4-cyano-3-methoxyphenyl)-N-(2,6-dimethylpyrimidin-4-yl)-2,5-dimethylpiperazine-1-carboxamide;
in another embodiment, (2R,5S)-4-(3-chloro-4-cyanophenyl)-N-(2-chloro-6-methylpyridin-4-yl)-2,5-dimethylpiperazine-1-carboxamide;
in another embodiment, (2R,5S)-4-(3-chloro-4-cyanophenyl)-N-(2-methoxy-6-methylpyridin-4-yl)-2,5-dimethylpiperazine-1-carboxamide;
in another embodiment, (2R,5S)-4-(3-bromo-4-cyanophenyl)-N-(2-methoxypyrimidin-5-yl)-2,5-dimethylpiperazine-1-carboxamide;
in another embodiment, (2R,5S)-4-(3-bromo-4-cyanophenyl)-N-(2-methoxy-6-methylpyridin-4-yl)-2,5-dimethylpiperazine-1-carboxamide ;

in another embodiment, (2R,5S)-4-(3-chloro-4-cyanophenyl)-2,5-dimethyl-N-[2-(piperidin-1-yl)pyrimidin-5-yl]piperazine-1-carboxamide.

Effects of the Present Invention

It was confirmed that a specific N-phenyl-(2R,5S)-dimethylpiperazine compound exhibits excellent antagonism against a mutant AR and has an excellent antitumor action in prostate cancer cells expressing the AR mutation. Accordingly, the present invention provides an excellent pharmaceutical composition for treating prostate cancer accompanying AR mutation, particularly, castration resistant prostate cancer (CRPC) resistant to bicalutamide or flutamide.

Embodiments for Carrying Out the Present Invention

Hereinafter, the present invention will be described in detail.
In the present specification, "AR" refers to an androgen receptor.
The "androgen receptor mutation" or "AR mutation" is a phenomenon in which mutation occurs in an amino acid sequence by the mutation of a gene encoding the protein of the androgen receptor due to various factors including drug administration, whereby the androgen receptor exhibits properties different from those of the natural androgen receptor. The "AR mutation" is, for example, "AR mutation" resulting from the use of anticancer drug. In an embodiment, it is "AR mutation" resulting from the use of bicalutamide or flutamide, and in another embodiment, it is "W741 mutation" or "T877 mutation". In another embodiment, it is "W741 mutation", and in still another embodiment, it is "W741C mutation" or "T877A mutation". In still another embodiment, it is "W741C mutation", and in still another embodiment, it is "T877 mutation". In still another embodiment, it is "T877A mutation".
The "W741 mutation" is a kind of androgen receptor mutation that was found when a patient administered with bicalutamide has developed AWS and is AR mutation in which a codon 741 of a natural AR gene expresses a mutation. An embodiment of the "W741 mutation" is "W741C mutation" or "W741L mutation", and in another embodiment it is "W741C mutation".
Among the above "W741 mutation", the "W741C mutation" is AR mutation in which a codon TGG encoding tryptophan has mutated to TGT encoding cysteine in the codon 741 of the natural AR gene.
Among the above "W741 mutation, the "W741 L mutation" is AR mutation in which the codon TGG encoding tryptophan has mutated to TTG encoding leucine in the codon 741 of the natural AR gene.
The "T877 mutation" is a kind of androgen receptor mutation that is expressed when a patient administered with flutamide has developed AWS, and AR mutation in which a codon 877 of the gene of natural AR expresses a mutation. An embodiment of the "T877 mutation" is "T877A mutation".
Among the above "T877 mutation", the "T877A mutation" results in a T877 mutant AR in which a codon ACT encoding threonine mutates to a codon GCT encoding alanine in the codon 877 of the natural AR gene.
The "bicalutamide resistance" is a state where AWS (Anti-androgen withdrawal syndrome) resulting from the administration of bicalutamide is confirmed, and the drug becomes ineffective for the prostate cancer. The main cause of the bicalutamide resistance is the W741C mutation.
The "flutamide resistance" is a state where AWS (Anti-androgen withdrawal syndrome) resulting from the administration of flutamide is confirmed, and the drug becomes ineffective for the prostate cancer. The main cause of the flutamide resistance is the T877A mutation.
The "Castration Resistant Prostate Cancer (CRPC)" is prostate cancer in which the pathological state advances even when the blood testosterone level is reduced to the level shown in a case of castration (orchiectomy) by hormone therapy or the like, and includes chemo-naive CRPC and chemo-failure CRPC.
The "chemo-naive CRPC" is castration resistant prostate cancer not treated with docetaxel.
The "chemo-failure CRPC" is castration resistant prostate cancer for which docetaxel becomes ineffective or of which the pathological state advances after treatment with docetaxel.
The compound which is an active ingredient of the pharmaceutical composition of the present invention includes other tautomers or optical isomers in some cases depending on the type of substituents. In the present specification, sometimes the compound is described merely in a single embodiment of those isomers, but the compound as an active ingredient of the pharmaceutical composition of the present invention includes those isomers as well as an isolate or mixture of the isomers. The compound which is an active ingredient of the pharmaceutical composition of the present invention includes all of the isomers.
In addition, the compound which is an active ingredient of the pharmaceutical composition of the present invention also includes pharmaceutically acceptable prodrugs thereof. The pharmaceutically acceptable prodrugs refer to compounds having a group that can be converted into an amino group, OH, $CO_2H$, and the like by solvolysis or under physiological conditions. Examples of groups forming the prodrugs include the groups disclosed in Prog. Med., 5, 2157-2161 (1985) or "Iyakuhin no Kaihatsu (Pharmaceutical research and development)" (Hirokawa Publishing Company, 1990), Vol. 7,

Bunshi Sekkei (Drug Design), 163-198.

Moreover, the compound which is an active ingredient of the pharmaceutical composition of the present invention sometimes forms an acid addition salt or a salt with a base depending on the type of substituents, and the salts are included in the present invention as long as they are pharmaceutically acceptable salts. Specific examples of the salts include acid addition salts with an inorganic acid such as hydrochloric acid, hydrobromic acid, hydriodic acid, sulfuric acid, nitric acid, phosphoric acid or the like or with an organic acid such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, aspartic acid, glutamic acid or the like, salts with an inorganic base such as sodium, potassium, magnesium, calcium, or aluminum, or with an organic base such as methylamine, ethylamine, ethanolamine, lysine, ornithine or the like, or ammonium salts.

Furthermore, the compound which is an active ingredient of the pharmaceutical composition of the present invention and pharmaceutically acceptable salts thereof include various hydrates or solvates and crystalline polymorphic substances. In addition, the compound which is an active ingredient of the pharmaceutical composition of the present invention and pharmaceutically acceptable salts thereof include compounds labeled with various radioactive or non-radioactive isotopes.

(Preparation process)

The compound which is an active ingredient of the pharmaceutical composition of the present invention and pharmaceutically acceptable salts thereof can be prepared by applying various known synthesis method by utilizing the characteristics based on the basic structure thereof or the type of substituents. At this time, depending on the type of functional group, sometimes it is effective to substituting beforehand the functional group into an appropriate protective group (a group that can be easily converted into the functional group) during the period from the stage of a starting material to the stage of an intermediate, in terms of the preparation technique. Examples of such a functional group include an amino group, a hydroxyl group, a carboxyl group, and the like, and examples of the protective group thereof include the protective groups disclosed in Wuts (P. G. M. Wuts) and Greene (T. W. Greene), "Greene's Protective Groups in Organic Synthesis (4th edition, 2006)". These may be used by being appropriately selected according to the reaction conditions. In this method, the protective group is introduced and causes a reaction, and then the protective group is optionally removed, whereby a desired compound can be obtained.

In addition, prodrugs of the compound which is an active ingredient of the pharmaceutical composition of the present invention can be prepared by introducing a specific group during the period from the stage of a starting material to the stage of an intermediate just like the above protective group, or by further causing a reaction by using the obtained compound. The reaction can be performed by applying methods known to a person skilled in the art, such as general esterification, amidation, dehydration and the like.

Hereinafter, a typical preparation process of the compound which is an active ingredient of the pharmaceutical composition of the present invention will be described. Each preparation process can be performed with reference to the reference document included in the corresponding description. Moreover, each preparation process is not limited to the following examples.

(General preparation process)

(Preparation process 1)

The compound which is an active ingredient of the pharmaceutical composition of the present invention can be prepared by a reaction among a compound (II), a compound (III), and a compound (IV) which is an activated carbonic acid derivative as a source of C=O. Herein, as an appropriate carbonic acid derivative, for example, triphosgene, phenyl chlorocarbonate, N,N'-carbonyldiimidazole (CDI), and those clearly known to a person skilled in the art can be used.

[Chem. 5]

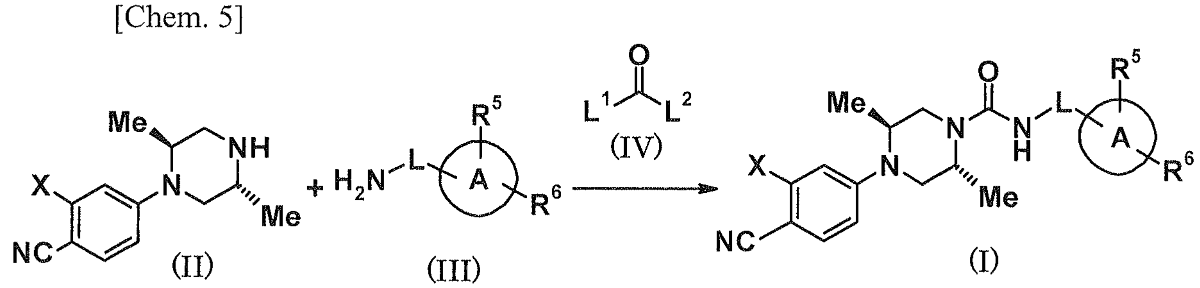

(The signs in the formula represent the following.
X: Cl, Br, methoxy, ethoxy, or isopropyloxy;
L: a bond or methylene;
Ring A: phenyl, pyridyl, pyrimidinyl, indolyl, quinolyl, or quinoxalyl;
$R^5$ and $R^6$: these are the same as or different from each other and represent $-CO-N(CH_3)_2$, F, methoxy, methyl, ethyl, or cyclopropyl;
here, the compound (I) represents any compound among example compounds Ex 1 to 22 and reference example compounds Ref 1-1 to 1-3 described later; and
$L^1$ and $L^2$ represent leaving groups.)
In this reaction, the compound (II) and the compound (III) are used in an equivalent amount, or one of them is used in an excessive amount. The mixture of these is stirred generally for 0.1 hours to 5 days under conditions ranging from cooling to heating preferably at -20°C to 60°C in a solvent inert to the reaction. Though not particularly limited, examples of the solvent used herein include aromatic hydrocarbons such as benzene, toluene, xylene and the like; halogenated hydrocarbons such as dichloromethane, 1,2-dichloroethane (DCE), chloroform and the like; ethers such as diethylether, tetrahydrofuran (THF), dioxane, dimethoxyethane and the like; N,N-dimethylformamide (DMF); dimethylsulfoxide; ethyl acetate; acetonitrile; water; and a mixture of these.

(Preparation process 2)

Moreover, the compound as an active ingredient of the pharmaceutical composition of the present invention can be prepared by reacting the compound (II) with a compound (VI) which is prepared from a compound (V) as a starting material.

[Chem. 6]

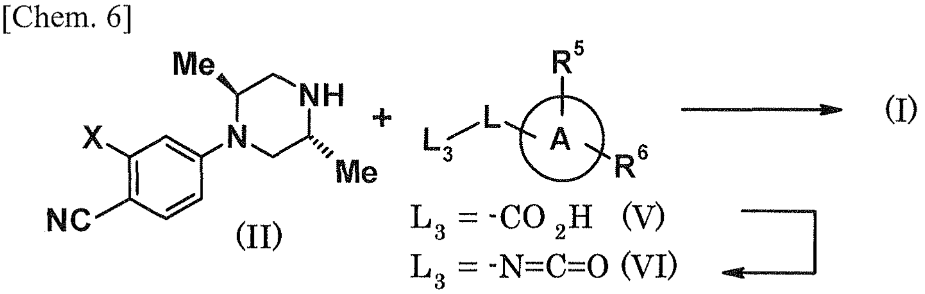


In this preparation process, the compound can be prepared by condensing the compound (II) with the compound (VI). Herein, the compound (VI) can be prepared by a rearrangement reaction in the reaction system after the compound (V) is subjected to azidation in advance by using diphenylphosphorylazide (DPPA) or another azidation agent.
In this preparation process, the condensation reaction following the rearrangement reaction is a technique well known to a person skilled in the art, and can be performed by applying the same reaction solvent and temperature as in the Preparation process 1. Specifically, the examples described later or Preparation process 1 of Patent Document 7 can be referred to.

(Starting material synthesis)

[Chem. 7]

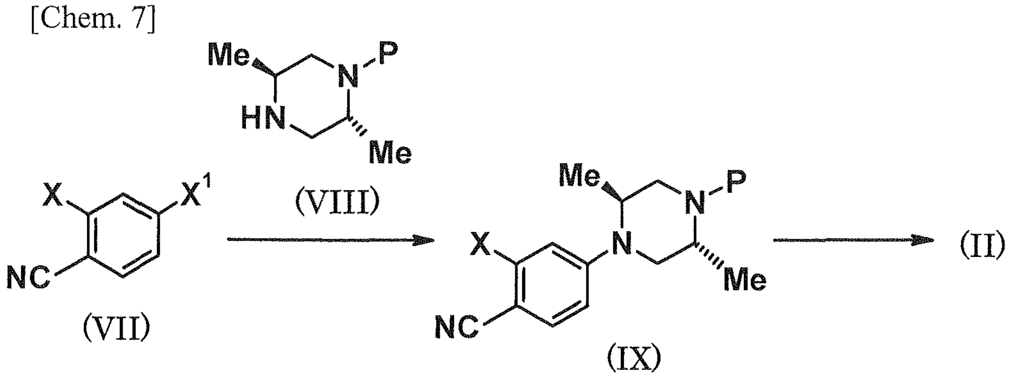

(VII) (VIII) (IX) (II)

(In the formula, $X^1$ represents a leaving group, and P represents a protective group of an amino group.)

The compound (II) can be prepared by performing deprotection after a substitution reaction between a compound (VII) and a compound (VIII).

Herein, examples of the leaving group $X^1$ include halogen, methanesulfonyloxy, p-toluenesulfonyloxy groups and the like, and in an embodiment, the leaving group $X^1$ represents F. Moreover, examples of the protective group P of an amino group include benzyl or t-butoxycarbonyl groups.

In this reaction, the compound (VII) and the compound (VIII) are used in an equivalent amount, or one of them is used in an excessive amount. The mixture of these is stirred generally for 0.1 hours to 5 days under conditions ranging from cooling to heating under reflux preferably at 0°C to 80°C in a solvent inert to the reaction or in the absence of a solvent. Though not particularly limited, examples of the solvent used herein include aromatic hydrocarbons such as benzene, toluene, xylene and the like; ethers such as diethylether, tetrahydrofuran, dioxane, dimethoxyethane and the like; halogenated hydrocarbons such as dichloromethane, 1,2-dichloroethane, chloroform and the like; N,N-dimethylformamide; dimethylsulfoxide; ethyl acetate; acetonitrile; and a mixture of these. Sometimes, it is advantageous to perform the reaction in the presence of an organic base such as triethylamine, N,N-diisopropylethylamine, N-methylmorpholine or the like, or an inorganic base such as potassium carbonate, sodium carbonate, potassium hydroxide or the like, in terms of causing the reaction to proceed smoothly.

The compound which is an active ingredient of the pharmaceutical composition of the present invention is isolated as a free compound, a salt thereof, a hydrate, a solvate, or a crystalline polymorphic substance, and purified. The salt of the compound can be prepared by a salt preparation reaction as a common method.

Isolation and purification can be performed by general chemical operations such as extraction, fractional crystallization, and various types of chromatographic fractionation.

Various isomers can be prepared by selecting appropriate starting compounds, or by using difference in physicochemical properties between the isomers. For example, the optical isomers are obtained by general optical resolution (for example, fractional crystallization for forming a diastereomer salt combined with an optically active salt or acid or chromatography using a chiral column or the like) for a racemic mixture. Alternatively, the optical isomers can also be prepared from appropriate starting compounds that are optically active.

The pharmaceutical composition of the present invention can be prepared using one or more active ingredients, a carrier or an excipient that is generally used for formulation, and other additives.

The composition can be administered in any forms such as oral administration by using a tablet, a pill, a capsule, granules, powder, or liquid, and parenteral administration by using an injection for intravenous injection, intramuscular injection, or the like, a suppository, eye drops, a transdermal agent, a transnasal agent, or an inhalation. The dose is appropriately determined case by case in consideration of the symptom, age and sex of the subject of administration, and the like. Generally, in the case of oral administration, the daily dosage for adult is about 0.001 mg/kg to 100 mg/kg which is administered once or administered 2 to 4 times in separate doses. In addition, when the composition is administered intravenously depending on the symptom, the daily dose for adult generally ranges from 0.0001 mg/kg to 10 mg/kg which is administered once to plural times. Moreover, in the case of inhalation, the daily dose for adult generally ranges from 0.001 mg/kg to 1 mg/kg which is administered once to plural times.

As the solid composition according to the present invention for oral administration, a tablet, powder, granules, and the like are used. In such a solid composition, one or more active substances are mixed with at least one inactive excipient, for example, lactic acid, mannitol, glucose, hydroxypropyl cellulose, microcrystalline cellulose, starch, polyvinyl pyrrolidone, or magnesium metasilicate aluminate. The composition may comprise inactive additives, for example, a lubricant such as magnesium stearate, a disintegrating agent such as sodium carboxymethyl starch, a solvent, and a solubilizing agent, according to the common method. The tablet or pill may optionally be coated with sugar or with a gastric or enteric coating agent.

The liquid composition for oral administration includes pharmaceutically acceptable emulsion, liquid, suspension, syrup, elixir, and the like, and comprises a generally used inactive solvent, for example, purified water or ethanol. The composition may comprise an adjuvant such as a solubilizer, a moisturizer, or a suspending agent, a sweetener, a flavoring agent, an aromatic, and a preservative, in addition to the inactive solvent.

The injection for parenteral administration includes sterile aqueous or non-aqueous liquid, suspension, and emulsion. Examples of the aqueous solvent include distilled water for injection and physiological saline. Examples of the non-aqueous solvent include propylene glycol, polyethylene glycol, plant oil such as olive oil, alcohols such as ethanol, Polysorbate 80 (trade name), and the like. The above composition may further comprise a tonicity agent, a preservative, a moisturizer, an emulsifier, a dispersant, a stabilizer, a solvent, and a solubilizing agent. These are sterilized by, for example, being filtered through a bacteria retentive filter, compounded with a bactericide, or irradiation. It is also possible to use these by preparing a sterile solid composition and then dissolving or suspending these in sterile water or a sterile solvent for injection before use.

The transmucosal agent such as an inhalation or transnasal agent is used in the form of solid, liquid, or semisolid, and can be prepared according to the method known in the related art. For example, an excipient such as lactose or starch, a pH adjustor, a preservative, a surfactant, a lubricant, a stabilizer, a thickener, and the like may be appropriately added thereto. For administration, an appropriate device for inhalation or insufflation can be used. For example, by using a known device such as a metered dose inhaler or an atomizer, the compound can be administered alone or administered as powder of a formulated mixture or as a solution or suspension by being combined with a pharmaceutically acceptable carrier. A dry powder inhaler and the like may be for single administration or multiple administration, and dry powder or powder-containing capsules can be used. Alternatively, the compound may be administered in the form of a pressurized aerosol spray using an appropriate ejection agent, for example, a suitable gas such as a chlorofluoroalkane, hydrofluoroalkane, or carbon dioxide.

Generally, in the case of oral administration, an appropriate daily dose is about 0.001 mg/kg to 100 mg/kg in terms of body weight, preferably 0.1 mg/kg to 30 mg/kg, and more preferably 0.1 mg/kg to 10 mg/kg, which is administered once or administered two to four times in separate doses. In the case of intravenous administration, an appropriate daily dose is about 0.0001 mg/kg to 10 mg/kg in terms of body weight, which is administered once or plural times a day in separate doses. In addition, the transmucosal agent is administered once to plural times a day in separate doses, in a dose of about 0.001 mg/kg to 100 mg/kg in terms of body weight. The dose is appropriately determined case by case in consideration of the symptoms, age, sex, and the like.

The pharmaceutical composition of the present invention comprises one or more kinds of the compound or a salt thereof as an active ingredient of the pharmaceutical composition of the present invention, in an amount of 0.01% by weight to 100% by weight, and 0.01 % by weight to 50% by weight as an embodiment, even though the amount varies with the route of administration, form of preparation, site of administration, and the type of excipient or additive.

The pharmaceutical composition of the present invention can be used concurrently with other treatment agent or preventive agent effective for the prostate cancer having a mutant AR. Herein, drugs inducing AR mutation are excluded from the above drugs. In the concurrent use, the composition and the agent may be administered simultaneously, administered sequentially one at a time, or administered at a desired time interval. The preparation for simultaneous administration may be a combination preparation or formulated individually.

## Examples

Hereinafter, based on examples, the preparation process of the compound as an active ingredient of the pharmaceutical composition of the present invention will be described in more detail, but the compound is not limited to the compounds described in the following examples. Moreover, the preparation process of starting compounds is described respectively in preparation examples, and compounds for being compared with the example compounds are described as reference example compounds. The reference example compounds are compounds having a N-phenyl-(2R, 5S)-dimethylpiperazine structure that the compound as an active ingredient of the pharmaceutical composition of the present invention also has, but the antagonistic action thereof against the mutant AR is greatly attenuated. The preparation process of the compound is not limited only to the preparation process of the specific examples described below. The compound can be prepared by a method as a combination of the preparation processes, or a method clearly known to a person skilled in the art. In addition, M represents [mol/L], ESI+ represents a m/z value in mass spectrometry (ionization ESI, $(M+H)^+$ unless otherwise specified), and EI+ represents $EI[M]^+$.

### Preparation example 1

To a mixture of potassium tert-butoxide (505 mg) and THF (5 ml) was added dropwise ethanol (0.264 ml) under ice bath cooling, followed by stirring at room temperature for 30 minutes. The reaction solution was cooled with an ice bath, and a mixture of 4-[(2S,5R)-2,5-dimethylpiperazin-1-yl]-2-fluorobenzonitrile (350 mg) and THF (3 ml) was added

dropwise thereto, followed by stirring at room temperature for 16 hours. Ethyl acetate (20 ml) and water (10 ml) were added to the reaction solution and the organic layer was separated. The organic layer was washed with saturated brine, dried over anhydrous sodium sulfate, and the solvent was evaporated under reduced pressure. The residue was purified by silica gel column chromatography (chloroform/methanol= 100/0 to 96/4), to afford 315 mg (81%) of 4-[(2S,5R)-2,5-dimethylpiperazin-1-yl]-2-ethoxybenzonitrile. ESI+:260

Preparation example 2

To a mixture of piperazin-2-one (5.06 g) and DMF (50 ml) were added triethylamine (8 ml) and 2-chloro-4-fluorobenzonitrile (8.1 g), followed by stirring at 80°C for a day. Water was added to the reaction solution, and the precipitated solids were collected by filtration and washed with water and diisopropylether, to afford 11.2 g (94%) of 2-chloro-4-(3-oxopiperazin-1-yl)benzonitrile. EI+: 235

Preparation example 3

To a mixture of 2-chloro-4-(3-oxopiperazin-1-yl)benzonitrile (11.2 g) and DMF (160 ml) was added sodium hydride (2.2 g, dispersed in 55% liquid paraffin), followed by stirring at room temperature for 10 minutes. Thereafter, benzyl bromide (6 ml) was added thereto, followed by stirring at room temperature for 1 hour. Water was added to the reaction solution, and the precipitated solids were collected by filtration and washed with water and diisopropylether, to afford 12.74 g (82%) of 4-(4-benzyl-3-oxopiperazin-1-yl)-2-chlorobenzonitrile. EI+: 325

Preparation example 4

A mixture of 4-(4-benzyl-3-oxopiperazin-1-yl)-2-chlorobenzonitrile (12.7 g), methyl iodide (7 ml), and THF (100 ml) was cooled to -78°C. To a mixture of diisopropylamine (16 ml) and THF (100 ml) was added dropwise n-butyllithium (40 ml, 2.77 M hexane solution) at -78°C, thereby preparing a lithium diisopropylamide (LDA) solution. The LDA solution was added to the above mixed solution over 40 minutes via cannula, followed by stirring again for 2 hours. A small amount of water was added to the reaction solution and allowed to warm up to room temperature, and the reaction solution was partitioned between ethyl acetate and water. The aqueous layer was extracted with ethyl acetate. The combined organic layer was washed with saturated brine, dried over anhydrous sodium sulfate, and concentrated under reduced pressure.
The residue was purified by silica gel column chromatography (hexane/ethyl acetate=10/0 to 5/5), to afford 6.14 g (44%) of 4-(4-benzyl-2,2-dimethyl-3-oxopiperazin-1-yl)-2-chlorobenzonitrile. EI+: 353

Preparation example 5

To a mixture of 4-(4-benzyl-2,2-dimethyl-3-oxopiperazin-1-yl)-2-chlorobenzonitrile (6.13 g) and THF (10 ml) were added 1,1,3,3-tetramethyldisiloxane (9.12 g) and hexachloroplatinic (IV) acid hexahydrate (0.8 g), followed by stirring at 50°C for 2 hours. After the insoluble material was removed from the reaction solution, the solvent was evaporated under reduced pressure, and the residue was purified twice by silica gel column chromatography (hexane/ethyl acetat=10/0 to 5/5), to afford 1.87 g (32%) of 4-(4-benzyl-2,2-dimethylpiperazin-1-yl)-2-chlorobenzonitrile. EI+: 339

Preparation example 6

To a mixture of 4-(4-benzyl-2,2-dimethylpiperazin-1-yl)-2-chlorobenzonitrile (1.87 g) and DCE (20 ml) was added 1-chloroethyl chloroformate (1 ml), followed by stirring with heating under reflux for 1 hour. After the reaction solution was left to cool, the solvent was evaporated under reduced pressure, and methanol (20 ml) was added to the residue, followed by stirring again for 1 hour with heating under reflux. The reaction solution was left to cool and concentrated under reduced pressure, and water and a small amount of 1 M hydrochloric acid were added thereto. The aqueous solution was washed with ethyl acetate, and the aqueous layer was basified by using a 1 M aqueous sodium hydroxide solution. Ethyl acetate was added thereto to perform liquid separation. The organic layer was washed with saturated brine and dried over anhydrous sodium sulfate, and then the solvent was evaporated under reduced pressure, to afford 1.31 g (95%) of 2-chloro-4-(2,2-dimethylpiperazin-1-yl)benzonitrile. EI+: 250

Preparation example 7

To a mixture of bis(trichloromethyl)carbonate (178 mg) and THF (6 ml) was added a mixture of 2-chloro-4-[(2S, 5R)-2,5-dimethylpiperazin-1-yl]benzonitrile (375 mg), triethylamine (0.314 ml), and THF (3 ml) under ice bath cooling,

followed by stirring for 1 hour at room temperature. Subsequently, the reaction solution was cooled with an ice bath, and a mixture of methyl 4-(aminomethyl)benzoate (273 mg), triethylamine (0.23 ml), and THF (3 ml) was added, followed by stirring again at room temperature for 3 hours. Saturated aqueous sodium hydrogen carbonate solution (10 ml), water (15 ml), and ethyl acetate (15 ml) were added to the reaction solution to perform liquid separation, and the organic layer was dried over anhydrous sodium sulfate, followed by evaporation of the solvent under reduced pressure. The residue was purified by silica gel column chromatography (chloroform/methanol=100/0 to 98/2), to afford 582 mg (88%) of methyl 4-[({[(2R,5S)-4-(3-chloro-4-cyanophenyl)-2,5-dimethylpiperazin-1-yl]carbonyl}amino)methyl]benzoate. EI+: 441

Preparation example 8

To a mixture of methyl 4-[({[(2R,5S)-4-(3-chloro-4-cyanophenyl)-2,5-dimethylpiperazin-1-yl]carbonyl}amino) methyl]benzoate (582 mg) and methanol/THF (1/1, 6 ml) was added a 1 M aqueous sodium hydroxide solution (3.3 ml), followed by stirring for 14 hours at room temperature. The reaction solution was concentrated under reduced pressure, and 1 M hydrochloric acid (3.3 ml) and water (10 ml) were added thereto under ice bath cooling. The precipitated solids were collected by filtration, to afford 512 mg (91%) of 4-[({[(2R,5S)-4-{3-chloro-4-cyanophenyl)-2,5-dimethylpiperazin-1-yl]carbonyl}amino)methyl]benzoic acid. EI+: 428

The structural formulae of the above preparation example compounds will be described in the tables described later.

Example 1

To a mixture of 4-[({[(2R,5S)-4-(3-chloro-4-cyanophenyl)-2,5-dimethylpiperazin-1-yl]carbonyl}amino)methyl] benzoic acid (256 mg) and DMF (3 ml) were added dimethylamine hydrochloride (147 mg), 1-(3-dimethylaminopropyl)-3-ethylcarbodiimide hydrochloride (WSC·HCl) (173 mg), 1-hydroxy-1H-benzotrizaole (HOBt) (41 mg), and triethylamine (0.251 ml) under ice bath cooling, followed by stirring for 18 hours at room temperature. The reaction solution was partitioned between added ethyl acetate (10ml) and water (10ml). The aqueous layer was extracted with ethyl acetate. The combined organic layer was washed with a saturated aqueous sodium hydrogen carbonate solution and saturated brine and dried over anhydrous sodium sulfate, and then the solvent was evaporated under reduced pressure. The residue was purified by silica gel column chromatography (chloroform/methanol=100/0 to 96/4), to afford 160 mg (59%) of (2R,5S)-4-(3-chloro-4-cyanophenyl)-N-[4-(dimethylcarbamoyl)benzyl]-2,5-dimethylpiperazine-1-carboxamide.

Example 6

To a mixture of 2-methoxypyrimidine-5-amine (120 mg) and pyridine (4 ml) was added phenyl chlorocarbonate (0.132 ml) under ice bath cooling, followed by stirring at room temperature for 1.5 hours. Thereafter, a mixture of 2-chloro-4-[(2S,5R)-2,5-dimethylpiperazin-1-yl]benzonitrile (200 mg) and pyridine (3 ml) was added thereto, followed by stirring at 100°C for 1 hour. After cooling, the reaction solution was concentrated under reduced pressure, and toluene (15 mlx2) was added to the residue and the azeotrope was evaporated. Ethyl acetate (20 ml) was added to the residue, followed by washing with a saturated aqueous sodium hydrogen carbonate solution and saturated brine and dried over anhydrous sodium sulfate. Then the solvent was evaporated under reduced pressure. The residue was purified by silica gel column chromatography (chloroform/methanol=100/0 to 96/4),to afford 258 mg (81%) of (2R,5S)-4-(3-chloro-4-cyanophenyl)-N-(2-methoxypyrimidin-5-yl)-2,5-dimethylpiperazine-1-carboxamide.

Example 16

To a mixture of 2-chloro-6-methylisonicotinic acid (151 mg) and ethyl acetate (3 ml) were added triethylamine (0.184 ml) and DPPA (0.208 ml), followed by stirring at room temperature for 2.5 hours. Toluene (15 ml) was added to the reaction solution, followed by washing with a saturated aqueous sodium hydrogen carbonate solution, dried over anhydrous sodium sulfate, and evaporation of the solvent under reduced pressure. The residue was dissolved in toluene (5 ml), stirred with heating under reflux for 40 minutes and then cooled to room temperature. A mixed solution of 2-chloro-4-[(2S,5R)-2,5-dimethylpiperazin-1-yl]benzonitrile (200 mg) and acetonitrile (3 ml) was added thereto, followed by stirring at room temperature for 1 hour. The precipitated solids were collected by filtration and washed with acetonitrile, to afford 235 mg (70%) of (2R,5S)-4-(3-chloro-4-cyanophenyl)-N-(2-chloro-6-methylpyridin-4-yl)-2,5-dimethylpiperazine-1-carboxamide.

Example 22

(2R,5S)-4-(3-chloro-4-cyanophenyl)-N-(2-cyclopropylpyrimidin-5-yl)-2,5-dimethylpiperazine-1-carboxamide

(compound A) (250 mg) which is the compound of Example 3-9 of Patent Document 3 was crystallized in 50% aqueous acetonitrile (6 ml) at room temperature, to give crystals of (2R,5S)-4-(3-chloro-4-cyanophenyl)-N-(2-cyclopropylpyrimidin-5-yl)-2,5-dimethylpiperazine-1-carboxamide monohydrate (111 mg) (44%).

Reference example 1-3

To a suspension of 2-chloro-4-[(2S,5R)-2,5-dimethylpiperazin-1-yl]benzonitrile (100 mg), methyl quinoxalin-2-yl carbamate (72 mg), and toluene (2 ml) was added alumina (80 mg), followed by stirring under reflux for a day. After cooling, the reaction solution was concentrated under reduced pressure, and the residue was purified by silica gel column chromatography (chloroform/methanol=100/0 to 95/5), and solidified by adding ethyl acetate and diisopropylether, to afford 34 mg (23%) of (2R,5S)-4-(3-chloro-4-cyanophenyl)-2,5-dimethyl-N-(quinoxalin-2-yl)piperazine-1-carboxamide as a solid.

By the same method as the method of the above examples, example compounds and reference example compounds described in the tables described later were prepared using respective starting materials corresponding thereto. In addition, the structural formula, physicochemical data, and preparation process of the example compounds and reference example compounds will be shown in the tables described later.

In the Examples, Preparation processes, Reference examples, and the following tables, the following abbreviations are used in some cases. Ex: Example compound number, PEx: Preparation example number, Ref: Reference example number, Str: Structural formula, Syn: Preparation process (indicating that the corresponding example compound is prepared by the same preparation process as the compound marked with an example number or reference example number), Dat: physicochemical data, ESI+: a m/z value in mass spectrometry (ionization ESI, $(M+H)^+$ unless otherwise specified), EI+: $EI[M]^+$, NMR: indicating 1H-NMR (400 MHz, DMSO-$d_6$) unless otherwise specified, Me: methyl, Et: ethyl, iPr: isopropyl

[Table 1]

| PEx | Str | | PEx | Str |
|---|---|---|---|---|
| 1 | 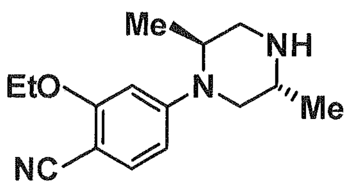 | | 4 | 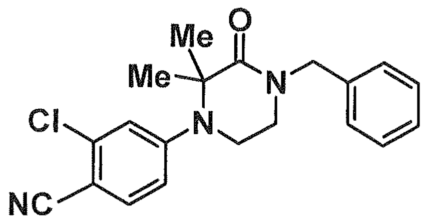 |
| 2 | 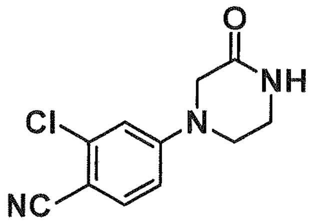 | | 5 | 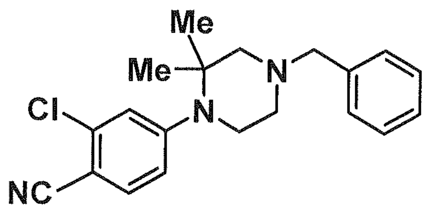 |
| 3 | 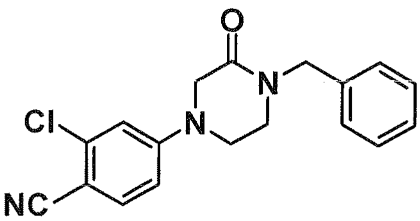 | | 6 | 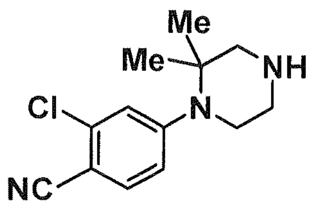 |

[Table 2]

| PEx | Str |
|---|---|
| 7 | 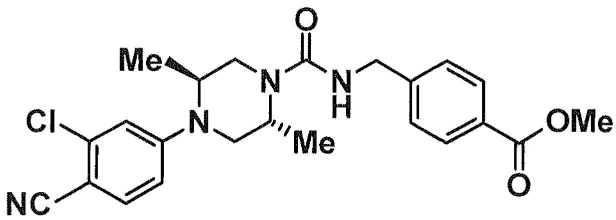 |
| 8 | 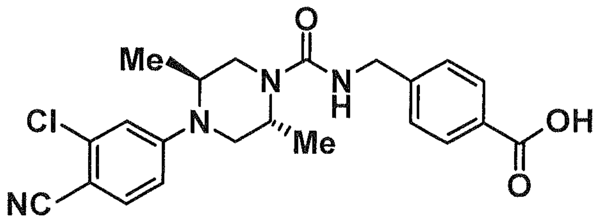 |

[Table 3]

| Ex | Str |
|---|---|
| 1 | 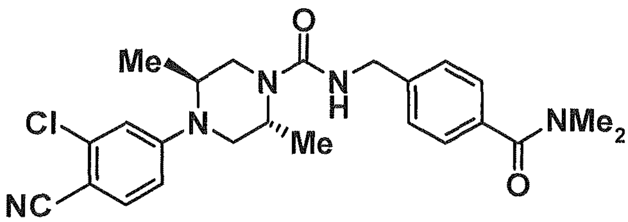 |
| 2 | 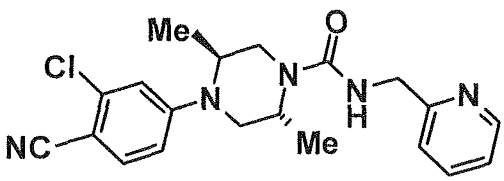 |
| 3 | 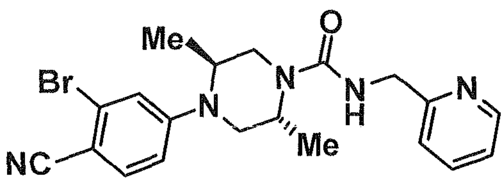 |
| 4 | 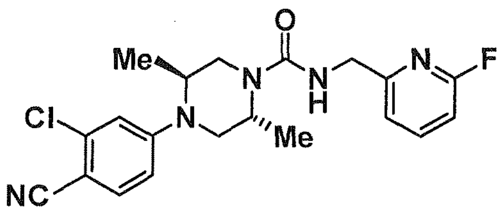 |
| 5 | 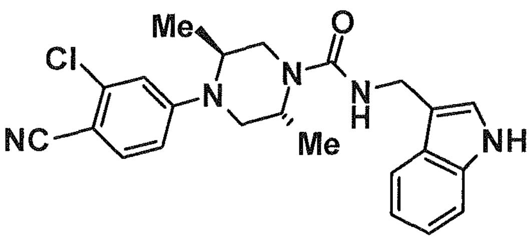 |

(continued)

| Ex | Str |
|---|---|
| 6 | 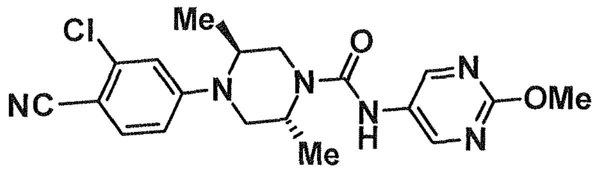 |

[Table 4]

| Ex | Str |
|---|---|
| 7 | 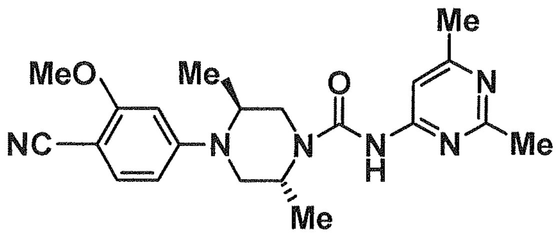 |
| 8 | 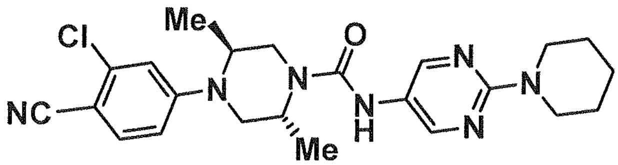 |
| 9 | 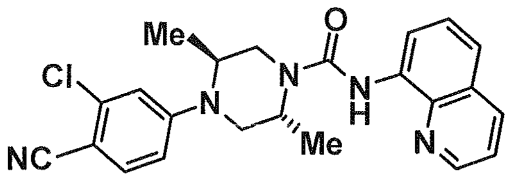 |
| 10 | 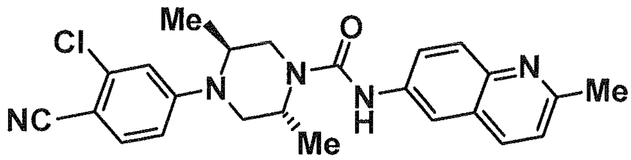 |
| 11 | 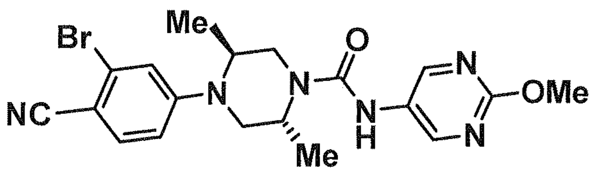 |
| 12 | 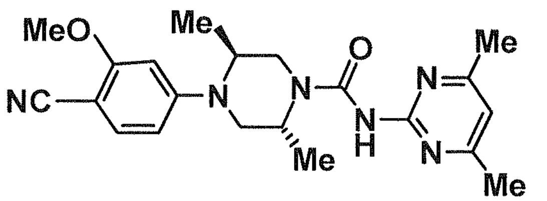 |

[Table 5]

| Ex | Str |
|---|---|
| 13 | 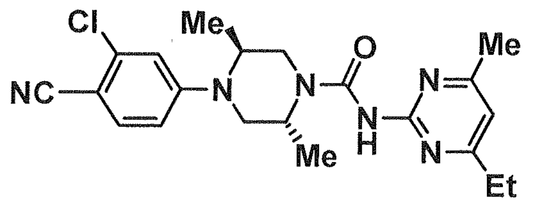 |
| 14 | 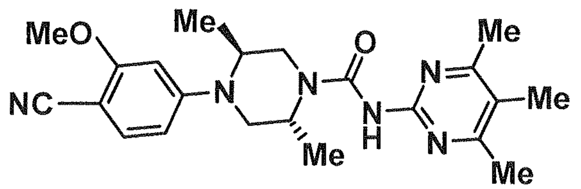 |
| 15 | 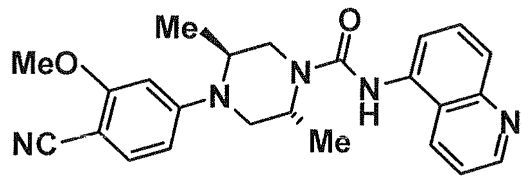 |
| 16 | 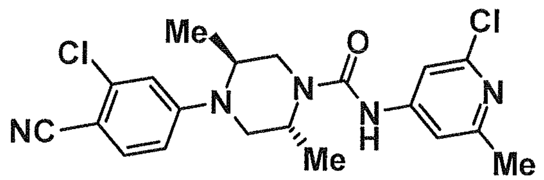 |
| 17 | 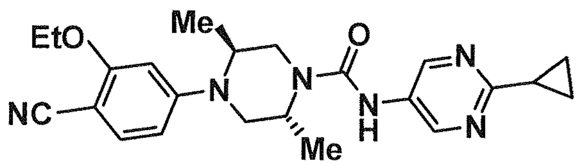 |
| 18 | 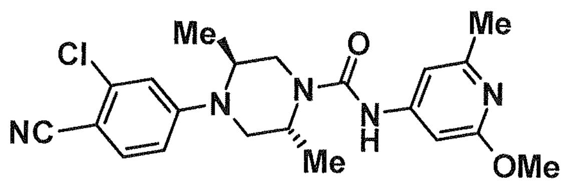 |

[Table 6]

| Ex | Str |
|---|---|
| 19 | 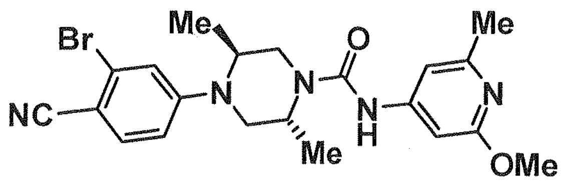 |
| 20 | 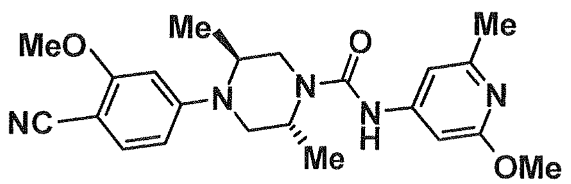 |

(continued)

| Ex | Str |
|---|---|
| 21 | 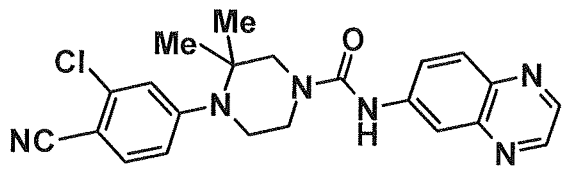 |
| 22 | 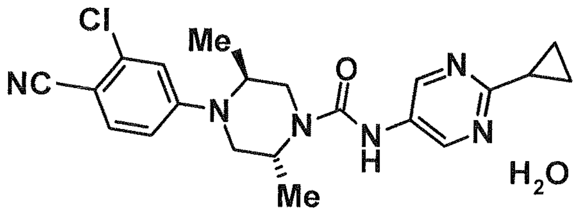 |

[Table 7]

| Ref | Str |
|---|---|
| 1-1 | 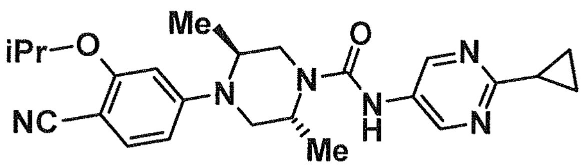 |
| 1-2 | 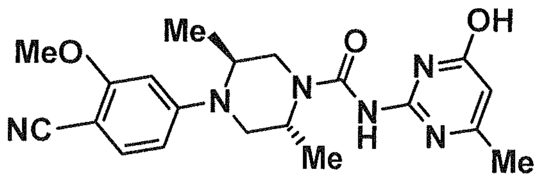 |
| 1-3 | 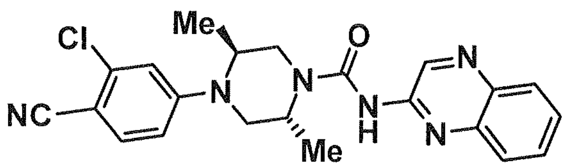 |

[Table 8]

| Ex | Syn | Dat |
|---|---|---|
| 1 | Ex1 | ESI+: 454 |
| 2 | PEx7 | ESI+: 384 |
| 3 | PEx7 | ESI+: 430, 428 |
| 4 | PEx7 | ESI+: 402 |
| 5 | PEx7 | ESI+: 424, 422 |
| 6 | Ex6 | ESI+: 401 |
| 7 | Ex6 | NMR: 1.05 (3H, d, J=6.4Hz), 1.18 (3H, d, J=6.4Hz), 2.33 (3H, s), 2.45 (3H, s), 3.25-3.45 (2H, m), 3.58 (1H, d, J=11.2Hz), 3.89 (3H, s), 3.9-4.0 (1H, m), 4.27 (1H, br), 4.52 (1H, br), 6.52 (1H, s), 6.56 (1H, d, J=8.9Hz), 7.42 (1H, d, J=8.8Hz), 7.51 (1H, s), 9.62 (1H, brs).<br>ESI+: 395 |

(continued)

| Ex | Syn | Dat |
|---|---|---|
| 8 | Ex6 | NMR: 1.09 (3H, d, J=6.5Hz), 1.16 (3H, d, J=6.7Hz), 1.45-1.65 (6H, m), 3.25-3.4 (2H,m), 3.6-3.7 (5H, m), 3.82 (1H, d, J=13.2Hz), 4.28 (1H, br), 4.42 (1H, br), 7.00 (1H, dd, J=2.4, 9.0Hz), 7.17 (1H, d, J=2.3Hz), 7.66 (1H, d, J=9.0Hz), 8.34 (2H, s), 8.39 (1H, brs).<br>ESI+: 454 |
| 9 | Ex6 | ESI+: 422, 420 |
| 10 | Ex6 | ESI+: 434 |
| 11 | Ex6 | ESI+: 445 |
| 12 | Ex6 | ESI+: 395 |
| 13 | Ex6 | ESI+: 415, 413 |
| 14 | Ex6 | ESI+: 409 |
| 15 | Ex6 | ESI+: 416 |

[Table 9]

| Ex | Syn | Dat |
|---|---|---|
| 16 | Ex16 | NMR:1.07 (3H, d, J=6.6Hz), 1.18 (3H, d, J=6.6Hz), 2.36 (3H, s), 3.25-3.45 (2H,m), 3.66 (1H, d, J=11.4Hz), 3.86 (1H, d, J=13.5Hz), 4.25 (1H, br), 4.47 (1H, br), 6.99 (1H, dd, J=2.5, 9.1Hz), 7.16 (1H, d, J=2.4Hz), 7.35 (1H, d, J=1.3Hz), 7.49 (1H, s), 7.66 (1H, d, J=9.0Hz), 9.08 (1H, brs).<br>ESI+: 418 |
| 17 | Ex16 | ESI+: 421 |
| 18 | Ex16 | ESI+: 416, 414 |
| 19 | Ex16 | ESI+: 460 |
| 20 | Ex16 | ESI+: 410 |
| 21 | Ex16 | ESI+: 423, 421 |
| 22 | Ex 22 | NMR:0.85-1.05(4H, m), 1.09(3H, d), 1.18(3H, d), 2.06-2.20(1H, m), 3.26-3.46(2H, m), 3.68(1H, d), 3.86(1H, d), 4.30(1H, br s), 4.47(1H, br s), 7.01(1H, dd), 7.18(1H, d), 7.67(1H, d), 8.72(2H, s), 8.78(1H, s).<br>ESI+: 411 |

[Table 10]

| Ref | Syn | Dat |
|---|---|---|
| 1-1 | Ex16 | ESI+: 435 |
| 1-2 | Ex6 | ESI+: 397 |
| 1-3 | Ref 1-3 | ESI+: 423,421 |

The pharmacological activity of the compound which is an active ingredient of the pharmaceutical composition of the present invention was confirmed by the following test.

Test example 1: Inhibitory action against transcriptional activation of human W741 C and W877A mutant ARs

CHO-K1 cells were transfected with a W741C or T877A mutant AR expression vector (pSG5-W741C-hAR or pcDNA3.1-T877A-hAR), thereby obtaining cells stably expressing human W741C and T877A mutant ARs. Moreover, these cells were also transfected with a luciferase reporter vector such that luciferase was expressed when the AR is activated, thereby obtaining cells stably expressing human W741C and T877A mutant ARs.

The CHO-K1 cells stably expressing the W741C or T877A mutant AR were seeded respectively in a 96-well luminoplate for cell culture by $2 \times 10^4$ cells and incubated overnight at 37°C, and then test compounds of various concentrations were added thereto simultaneously with DHT (final concentration 0.3 nM). Subsequently, the cells were incubated overnight again at 37°C and then treated with a luciferase assay system. The amount of light emitted from the cells was measured using a luminometer and employed as luciferase activity resulting from the transcriptional activation of the W741C or T877A mutant AR.
The transcriptional activation inhibitory action of the compound which is an active ingredient of the pharmaceutical composition of the present invention was evaluated as the luciferase inhibitory activity. An $IC_{50}$ value was calculated by Sigmoid-Emax model nonlinear regression analysis.
As the test compounds of the above test, bicalutamide, Hydroxyflutamide, the compounds which is active ingredients of the present invention (Compound A (Patent Document 3 and Examples 3 to 9) and compounds of Examples 7, 8, 11, 16, 18, 19, and 22), and Reference examples 1-1 to 1-3 were used, and the inhibitory action against the transcriptional activation of the respective receptors including a wild type AR (Wild), the W741C mutant AR, and the T877A mutant AR was evaluated. The inhibitory action of the respective test compounds against the receptor was calculated as an $IC_{50}$ value, and the values are described in the following table. In addition, "-" means that the test was not performed.
[Table 11]

| | Wild | W741C | T877A |
|---|---|---|---|
| **Bicalutamide** | **230 nM** | **>1000 nM** | **600 nM** |
| **Hydroxyflutamide** | **32 nM** | **61 nM** | **>1000 nM** |
| **Compound A** | **23 nM** | **56 nM** | **20 nM** |
| **Ex 7** | **76 nM** | **32 nM** | **-** |
| **Ex 8** | **17 nM** | **66 nM** | **-** |
| **Ex 11** | **26 nM** | **120 nM** | **23 nM** |
| **Ex 16** | **45 nM** | **96 nM** | **24 nM** |
| **Ex 18** | **10 nM** | **17 nM** | **15 nM** |
| **Ex 19** | **11 nM** | **26 nM** | **19 nM** |
| **Ex 22** | **35 nM** | **87 nM** | **39 nM** |
| **Ref 1-1** | **140 nM** | **>1000 nM** | **350 nM** |
| **Ref 1-2** | **58 nM** | **650 nM** | **89 nM** |
| **Ref 1-3** | **51 nM** | **560 nM** | **680 nM** |

Hereinafter, the contents of the above table will be described.
In the table, >1000 nM of $IC_{50}$ value means that the inhibitory action against transcriptional activation is not observed, and the antagonistic activity against AR is practically ineffective.
It was confirmed that bicalutamide, which is an existing AR antagonist, does not exhibit the inhibitory action against transcriptional activation causing W741C mutation which is a characteristic of bicalutamide resistance, and the antagonistic activity thereof against the W741C mutant AR receptor is practically ineffective.
It was confirmed that the Hydroxyflutamide is an active metabolite of flutamide, and the antagonistic activity thereof against the AR receptor showing A877A mutation which is a characteristic of flutamide resistance is practically ineffective.
It was confirmed that the Compound A and the compounds of Examples 7, 8, 11, 16, 18, 19, and 22 that are compounds which is active ingredients of the pharmaceutical composition of the present invention have the inhibitory action against transcriptional activation of any of ARs including the wild type AR, W741C mutant AR, and A877A mutant AR, and have the antagonistic activity against AR.
It was confirmed that the compounds of Reference examples 1-1, 1-2, and 1-3 have the inhibitory action against transcriptional activation of the wild type AR and the antagonistic activity against the wild type AR. However, it was confirmed that the inhibitory action of the compounds against transcriptional activation of either or both of W741C and W877A mutant ARs is greatly attenuated, and the antagonistic activity against the mutant AR is also attenuated, compared to the compound A and the compounds of Examples 7, 8, 11, 16, 18, 19, and 22.
Moreover, Example 22 (Ex 22) is a monohydrate of the compound A and is not disclosed or suggested in Patent Document

3.

As shown in the Table 11, it was confirmed that even among the AR antagonist compounds which have the similar chemical structure and are effective for the wild type AR, some of them are effective for the mutant AR while others are not. That is, among the N-phenyl-(2R,5S)-dimethylpiperazine derivatives effective for the wild type AR, the activity thereof against the mutant AR greatly varies with the individual substituent, and this is extremely unexpected.

Test example 2: Antitumor action in KUCaP cancer-bearing mouse

KUCaP is a human prostate cancer cell established by The University of Kyoto (Cancer Res 2005; 65: 9611-9616), which has a W741C mutant AR and can be subcultured in vivo (mouse). A fragment of the cell was subcutaneously grafted into the back of male SCID mice. At the point in time when the tumor volume became about 200 $mm^3$ to 400 $mm^3$, the animals were grouped (5 per group) such that each group had the same tumor volume, and the administration of the test compound was started. The test compound was dissolved in 25% propylene glycol/25% Tween 80/50% purified water, and orally administered two times a day at a dose of 5 mg/kg/10 mL (10 mg/kg/day) for 14 days. In addition, to the control group, 25% propylene glycol/25% Tween 80/50% purified water were administered at a dose of 5 mL/kg. To determine the tumor size, a Vernier caliper was used to measure a major axis (mm) and a minor axis (mm) of the tumor, and a tumor volume ($mm^3$) was calculated by a calculation formula "major axis$\times$minor axis$^2\times$0.5". In addition, an inhibition rate [%] of the test drug was calculated by the following calculation formula. Inhibition rate [%] =100$\times${1-[(tumor volume of test compound group on the 14$^{th}$ day - tumor volume of test compound group on the starting date of administration]/[tumor volume of control group on the 14$^{th}$ day - tumor volume of control group on the starting date of administration)]}

The following can be referred to as a reference document of Test example 2.

Yoshida T, Kinoshita H, Segawa T et al. "Antiandrogen bicalutamide promotes tumor growth in a novel androgen-dependent prostate cancer xenograft model derived from bicalutamide-treated patient" Cancr Res 2005; 65: 9611-9616

According to the Test example 2, some compounds which is active ingredients of the pharmaceutical composition of the present invention were evaluated. The inhibition rate [%] thereof is shown in the following table. In addition, in the table, Ex indicates the Example compound number.

[Table 12]

| Compound | Inhibition(%) |
|---|---|
| A | 100 |
| Ex 7 | 50 |
| Ex 8 | 32 |
| Ex 11 | 95 |
| Ex 16 | 83 |
| Ex 19 | 73 |
| Ex 18 | 73 |
| Ex 22 | 100 |

Test example 3: evaluation of binding activity of human W741C and T877A mutant androgen receptors (ARs)

CHO-K1 cells were transfected with a W741C or T877A mutant AR expression vector (pSG5-W741C-hAR or pcDNA 3.1-T877A-hAR), whereby cells forced to transiently express human W741C and T877A mutant ARs were obtained. These cells were seeded in a 24-well plate at $5\times10^4$ cell/well and incubated overnight at 37°C. The medium was removed and the test compound diluted with a medium supplemented with DCC-FBS and [$^3$H]DHT were added thereto, followed by culturing for 4 hours at 37°C. After the medium was removed, a lysis buffer was added thereto to cause lysis of the cells, and radioactivity of the supernatant was measured. From the value of radioactivity, $IC_{50}$ of the inhibitory activity of the test compound against the specific binding of [$^3$H]DHT was obtained.

Test example 4: Action on the growth of W741C mutant AR-expressing human prostate cancer cell line LNCaP (W741C-hAR-LNCaP)

1) Obtaining W741C-hAR-LNCaP

LNCaP cells which are a human prostate cancer cell line were transfected with a W741C mutant AR expression vector (pSG5-W741C-hAR), whereby W741C-hAR-LNCaP was obtained.

2) W741 C-hAR-LNCaP growth-promoting action

The W741C-hAR-LNCaP cells were seeded at $5\times10^3$ cell/well into a 96-well plate coated with Poly-L-lysine and incubated for a day, and a compound diluted with a medium supplemented with DCC-FBS and DHT or a solvent (DMSO) were added thereto, followed by incubation again. After 7 days, the amount of protein in each well was measured by sulforhodamine B assay (see the document described later). If the amount of protein increased more than the amount of protein in a well containing only a solvent (without the test compound), this was evaluated as growth promoting action of the compound.

3) W741C-hAR-LNCaP growth-inhibiting action

The W741C-hAR-LNCaP cells were seeded at $5\times10^3$ cell/well into a 96-well plate coated with Poly-L-lysine and incubated for a day, and both the test compound (or a solvent) diluted with a medium supplemented with DCC-FBS and DHT (1 nM) were added thereto, followed by incubation again. After 7 days, the amount of protein in each well was measured by sulforhodamine B assay. The inhibition rate (%) was calculated by the following formula

$$\text{Inhibition rate (\%)}=100[(I-B)-(X-B)]/(I-B)$$

I: the amount of protein obtained when only 1 nM of DHT is added
B: the amount of protein obtained when only a solvent is added
X: the amount of protein obtained when the present compound and 1 nM of DHT are added simultaneously

As the reference document relating to the sulforhodamine B assay in Test example 4, the following Reference documents 1 and 2 can be referred to.
Reference document 1: Skehan P, Storeng R, Scudiero D et al. New colorimetric cytotoxicity assay for anticancer-drug screening. J Natl Cancer Inst 1990; 82: 1107-1112
Reference document 2: Papazisis KT, Geromichalos GD, Dimitriadis KA et al. Optimization of the sulforhodamine B colorimetric assay. J Immunol Methods 1997; 208: 151-158
From the results of Test examples 1 and 2, it was confirmed that the compounds which is active ingredients of the pharmaceutical composition of the present invention have an inhibitory action against transcriptional activation of W741C and W877A mutant ARs, and an antitumor action in a mouse to which the human prostate cancer cell having the W741C mutant AR has been grafted. Accordingly, this shows that the compounds which are active ingredients of the pharmaceutical composition of the present invention are useful for treating AR-related diseases accompanying AR mutation, particularly, for treating prostate cancer accompanying AR mutation.

Industrial Applicability

A compound which is an active ingredient of the pharmaceutical composition of the present invention or a pharmaceutically acceptable salt thereof exhibits excellent antagonistic activity against a mutant AR and has an excellent antitumor action in an animal model of prostate cancer accompanying the AR mutation. Thus, the present invention is useful as a therapeutic agent of prostate cancer accompanying the AR mutation.

## Claims

**1.** A pharmaceutical composition for treating castration resistant prostate cancer, which comprises, as an active ingredient, a compound or a pharmaceutically acceptable salt thereof selected from a group consisting of (2R,5S)-4-(3-chloro-4-cyanophenyl)-N-(2-cyclopropylpyrimidin-5-yl)-2,6-dimethylpiperazine-1-carboxamide,

(2R,5S)-4-(4-cyano-3-methoxyphenyl)-N-(2,6-dimethylpyrimidin-4-yl)-2,5-dimethylpiperazine-1-carboxamide,
(2R,5S)-4-(3-chloro-4-cyanophenyl)-N-(2-chloro-6-methylpyridin-4-yl)-2,5-dimethylpiperazine-1-carboxamide,
(2R,5S)-4-(3-chloro-4-cyanophenyl)-N-(2-methoxy-6-methylpyridin-4-yl)-2,5-dimethylpiperazine-1-carboxamide,
(2R,5S)-4-(3-bromo-4-cyanophenyl)-N-(2-methoxypyrimidin-5-yl)-2,5-dimethylpiperazine-1-carboxamide,
(2R,5S)-4-(3-bromo-4-cyanophenyl)-N-(2-methoxy-6-methylpyridin-4-yl)-2,5-dimethylpiperazine-1-carboxamide,
and
(2R,5S)-4-(3-chloro-4-cyanophenyl)-2,5-dimethyl-N-[2-(piperidin-1-yl)pyrimidin-5-yl]piperazine-1-carboxamide,
and
pharmaceutically acceptable salts thereof.

2. The pharmaceutical composition according to Claim 1, wherein the castration resistant prostate cancer is castration resistant prostate cancer resistant to bicalutamide.

3. The pharmaceutical composition according to Claim 1, wherein the castration resistant prostate cancer is castration resistant prostate cancer resistant to flutamide.

4. A compound or a pharmaceutically acceptable salt thereof selected from a group consisting of (2R,5S)-4-(3-chloro-4-cyanophenyl)-N-(2-cyclopropylpyrimidin-5-yl)-2,5-dimethylpiperazine-1-carboxamide monohydrate,
(2R,5S)-4-(4-cyano-3-methoxyphenyl)-N-(2,6-dimethylpyrimidin-4-yl)-2,5-dimethylpiperazine-1-carboxamide,
(2R,5S)-4-(3-chloro-4-cyanophenyl)-N-(2-chloro-6-methylpyridin-4-yl)-2,5-dimethylpiperazine-1-carboxamide,
(2R,5S)-4-(3-chloro-4-cyanophenyl)-N-(2-methoxy-6-methylpyridin-4-yl)-2,5-dimethylpiperazine-1-carboxamide,
(2R,5S)-4-(3-bromo-4-cyanophenyl)-N-(2-methoxypyrimidin-5-yl)-2,5-dimethylpiperazine-1-carboxamide,
(2R,5S)-4-(3-bromo-4-cyanophenyl)-N-(2-methoxy-6-methylpyridin-4-yl)-2,5-dimethylpiperazine-1-carboxamide,
and
(2R,5S)-4-(3-chloro-4-cyanophenyl)-2,5-dimethyl-N-[2-(piperidin-1-yl)pyrimidin-5-yl]piperazine-1-carboxamide,
and
pharmaceutically acceptable salts thereof.

5. The compound or a pharmaceutically acceptable salt thereof according to Claim 4, wherein the compound is (2R,5S)-4-(3-chloro-4-cyanophenyl)-N-(2-cyclopropylpyrimidin-5-yl)-2,5-dimethylpiperazine-1-carboxamide monohydrate.

6. A compound or a pharmaceutically acceptable salt thereof for treating castration resistant prostate cancer, which is selected from a group consisting of (2R,5S)-4-(3-chloro-4-cyanophenyl)-N-(2-cyclopropylpyrimidin-5-yl)-2,5-dimethylpiperazine-1-carboxamide,
(2R,5S)-4-(4-cyano-3-methoxyphenyl)-N-(2,6-dimethylpyrimidin-4-yl)-2,5-dimethylpiperazine-1-carboxamide,
(2R,5S)-4-(3-chloro-4-cyanophenyl)-N-(2-chloro-6-methylpyridin-4-yl)-2,5-dimethylpiperazine-1-carboxamide,
(2R,5S)-4-(3-chloro-4-cyanophenyl)-N-(2-methoxy-6-methylpyridin-4-yl)-2,5-dimethylpiperazine-1-carboxamide,
(2R,5S)-4-(3-bromo-4-cyanophenyl)-N-(2-methoxypyrimidin-5-yl)-2,5-dimethylpiperazine-1-carboxamide,
(2R,5S)-4-(3-bromo-4-cyanophenyl)-N-(2-methoxy-6-methylpyridin-4-yl)-2,5-dimethylpiperazine-1-carboxamide,
and
(2R,5S)-4-(3-chloro-4-cyanophenyl)-2,5-dimethyl-N-[2-(piperidin-1-yl)pyrimidin-5-yl]piperazine-1-carboxamide,
and
pharmaceutically acceptable salts thereof.

7. Use of a compound or a pharmaceutically acceptable salt thereof for manufacturing a pharmaceutical composition for treating castration resistant prostate cancer, wherein the compound or a pharmaceutically acceptable salt thereof is selected from a group consisting of (2R,5S)-4-(3-chloro-4-cyanophenyl)-N-(2-cyclopropylpyrimidin-5-yl)-2,5-dimethylpiperazine-1-carboxamide, (2R,5S)-4-(4-cyano-3-methoxyphenyl)-N-(2,6-dimethylpyrimidin-4-yl)-2,5-dimethylpiperazine-1-carboxamide,
(2R,5S)-4-(3-chloro-4-cyanophenyl)-N-(2-chloro-6-methylpyridin-4-yl)-2,5-dimethylpiperazine-1-carboxamide,
(2R,5S)-4-(3-chloro-4-cyanophenyl)-N-(2-methoxy-6-methylpyridin-4-yl)-2,5-dimethylpiperazine-1-carboxamide,
(2R,5S)-4-(3-bromo-4-cyanophenyl)-N-(2-methoxypyrimidin-5-yl)-2,5-dimethylpiperazine-1-carboxamide,
(2R,5S)-4-(3-bromo-4-cyanophenyl)-N-(2-methoxy-6-methylpyridin-4-yl)-2,5-dimethylpiperazine-1-carboxamide,
and
(2R,5S)-4-(3-chloro-4-cyanophenyl)-2,5-dimethyl-N-[2-(piperidin-1-yl)pyrimidin-5-yl]piperazine-1-carboxamide,
and
pharmaceutically acceptable salts thereof.

8. Use of a compound or a pharmaceutically acceptable salt thereof for treating castration resistant prostate cancer, wherein the compound or a pharmaceutically acceptable salt thereof is selected from a group consisting of (2R,5S)-4-(3-chloro-4-cyanophenyl)-N-(2-cyclopropylpyrimidin-5-yl)-2,5-dimethylpiperazine-1-carboxamide, (2R,5S)-4-(4-cyano-3-methoxyphenyl)-N-(2,6-dimethylpyrimidin-4-yl)-2,5-dimethylpiperazine-1-carboxamide,
(2R,5S)-4-(3-chloro-4-cyanophenyl)-N-(2-chloro-6-methylpyridin-4-yl)-2,5-dimethylpiperazine-1-carboxamide,
(2R,5S)-4-(3-chloro-4-cyanophenyl)-N-(2-methoxy-6-methylpyridin-4-yl)-2,5-dimethylpiperazine-1-carboxamide,
(2R,5S)-4-(3-bromo-4-cyanophenyl)-N-(2-methoxypyrimidin-5-yl)-2,5-dimethylpiperazine-1-carboxamide,
(2R,5S)-4-(3-bromo-4-cyanophenyl)-N-(2-methoxy-6-methylpyridin-4-yl)-2,5-dimethylpiperazine-1-carboxamide, and
(2R,5S)-4-(3-chloro-4-cyanophenyl)-2,5-dimethyl-N-[2-(piperidin-1-yl)pyrimidin-5-yl]piperazine-1-carboxamide, and
pharmaceutically acceptable salts thereof.

9. A method of treating castration resistant prostate cancer, which comprises administering to a subject an effective amount of a compound or a pharmaceutically acceptable salt thereof selected from a group consisting of (2R,5S)-4-(3-chloro-4-cyanophenyl)-N-(2-cyclopropylpyrimidin-5-yl)-2,5-dimethylpiperazine-1-carboxamide,
(2R,5S)-4-(4-cyano-3-methoxyphenyl)-N-(2,6-dimethylpyrimidin-4-yl)-2,5-dimethylpiperazine-1-carboxamide,
(2R,5S)-4-(3-chloro-4-cyanophenyl)-N-(2-chloro-6-methylpyridin-4-yl)-2,5-dimethylpiperazine-1-carboxamide,
(2R,5S)-4-(3-chloro-4-cyanophenyl)-N-(2-methoxy-6-methylpyridin-4-yl)-2,5-dimethylpiperazine-1-carboxamide,
(2R,5S)-4-(3-bromo-4-cyanophenyl)-N-(2-methoxypyrimidin-5-yl)-2,5-dimethylpiperazine-1-carboxamide,
(2R,5S)-4-(3-bromo-4-cyanophenyl)-N-(2-methoxy-6-methylpyridin-4-yl)-2,5-dimethylpiperazine-1-carboxamide, and
(2R,5S)-4-(3-chloro-4-cyanophenyl)-2,5-dimethyl-N-[2-(piperidin-1-yl)pyrimidin-5-yl]piperazine-1-carboxamide, and
pharmaceutically acceptable salts thereof.

## INTERNATIONAL SEARCH REPORT

International application No.
PCT/JP2011/074261

A. CLASSIFICATION OF SUBJECT MATTER
*C07D401/12*(2006.01)i, *A61K31/496*(2006.01)i, *A61K31/506*(2006.01)i, *A61P5/26*(2006.01)i, *A61P35/00*(2006.01)i, *C07D403/12*(2006.01)i

According to International Patent Classification (IPC) or to both national classification and IPC

B. FIELDS SEARCHED

Minimum documentation searched (classification system followed by classification symbols)
C07D401/12, A61K31/496, A61K31/506, A61P5/26, A61P35/00, C07D403/12

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
REGISTRY(STN), CAPLUS(STN)

C. DOCUMENTS CONSIDERED TO BE RELEVANT

| Category* | Citation of document, with indication, where appropriate, of the relevant passages | Relevant to claim No. |
|---|---|---|
| Y | WO 2004/007471 A1 (Yamanouchi Pharmaceutical Co., Ltd.), 22 January 2004 (22.01.2004), page 8, lines 19 to 20; examples 3-9, 3-21, 3-27; test method 1-3; claims 1 to 8 & JP 4449746 B & US 2005/0261303 A1 & US 2008/0214543 A1 & EP 1557411 A1 | 1-7 |
| Y | JP 2001-328938 A (Yamanouchi Pharmaceutical Co., Ltd.), 27 November 2001 (27.11.2001), claims 1 to 3; examples 18-10, 18-23, 19-1 (Family: none) | 1-7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>17 November, 2011 (17.11.11) | Date of mailing of the international search report<br>29 November, 2011 (29.11.11) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2011/074261

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2005/040136 A1 (BRISTOL MYERS SQUIBB CO.), 06 May 2005 (06.05.2005), page 38, line 20 to page 39, line 12 & US 2005/0124625 A1 | 1-7 |
| Y | Jounal of Cellular Biochemistry, 2004, Vol.91, pp3-12 | 1-7 |
| Y | WO 2009/028543 A1 (Takeda Chemical Industries, Ltd.), 05 March 2009 (05.03.2009), paragraphs [0014], [0110] & US 2010/0227846 A1 & US 2009/0270359 A1 & EP 2194045 A1 | 1-7 |
| Y | WO 2009/059077 A1 (BRISTOL-MYERS SQUIBB CO.), 07 May 2009 (07.05.2009), paragraph [0006], [00111] & US 2010/0256180 A1 | 1-7 |

Form PCT/ISA/210 (continuation of second sheet) (July 2009)

**INTERNATIONAL SEARCH REPORT**

International application No.
PCT/JP2011/074261

**Box No. II Observations where certain claims were found unsearchable (Continuation of item 2 of first sheet)**

This international search report has not been established in respect of certain claims under Article 17(2)(a) for the following reasons:

1. [X] Claims Nos.: 8, 9
because they relate to subject matter not required to be searched by this Authority, namely:
Claims 8 and 9 pertain to methods for treatment of human being by therapy and thus relate to a subject matter on which this International Searching Authority is not required to carry out a search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005040136 A
- WO 2000017163 A
- WO 2004007471 A
- US 20050159468 A1
- WO 2006064944 A
- WO 2009028543 A
- WO 2009059077 A
- WO 2009003077 A


### Non-patent literature cited in the description


- *J. Cell. Biochem,* 2007, vol. 91, 3-12
- *Cancer Research,* 2003, vol. 63, 149-153
- *Journal of Cellular Biochemistry,* 2004, vol. 91, 3-12
- *Cancer Research,* 2002, vol. 62, 1496-1502
- *Science,* 2009, vol. 324, 787-790
- *Prog. Med.,* 1985, vol. 5, 2157-2161
- Iyakuhin no Kaihatsu. Bunshi Sekkei. Hirokawa Publishing Company, 1990, vol. 7, 163-198
- **WUTS ; GREENE.** Greene's Protective Groups in Organic Synthesis. 2006
- *Cancer Res,* 2005, vol. 65, 9611-9616
- **YOSHIDA T ; KINOSHITA H ; SEGAWA T et al.** Antiandrogen bicalutamide promotes tumor growth in a novel androgen-dependent prostate cancer xenograft model derived from bicalutamide-treated patient. *Cancr Res,* 2005, vol. 65, 9611-9616
- **SKEHAN P ; STORENG R ; SCUDIERO D et al.** New colorimetric cytotoxicity assay for anticancer-drug screening. *J Natl Cancer Inst,* 1990, vol. 82, 1107-1112
- **PAPAZISIS KT ; GEROMICHALOS GD ; DIMITRIADIS KA et al.** Optimization of the sulforhodamine B colorimetric assay. *J Immunol Methods,* 1997, vol. 208, 151-158